# EUROPEAN PATENT APPLICATION

(11) **EP 3 403 511 A1**
(43) Date of publication of application: **21.11.2018**
(21) Application number: 16885180.6
(22) Date of filing: 07.03.2016

(54) **FOOD COMPOSITION FOR PREVENTING AND ALLEVIATING FEMALE MENOPAUSAL SYMPTOMS, CONTAININGVITIS VINIFERA**

(30) Priority: 12.01.2016 KR 20160003956
(71) Applicant: Frombio Co., Ltd, Jeju-si, Jeju-do 63309 (KR)
(72) Inventor: KIM, Ji Hoon, Jeju-si Jeju-do 63343 (KR); SHIM, Tae Jin, Suwon-si Gyeonggi-do 16663 (KR)
(74) Representative: Rupp, Christian
(86) International application number: PCT/KR2016/002224
(87) International publication number: WO 2017/122868

(57) **Abstract**

Provided is a food composition for preventing and alleviating female menopause symptoms containing a grape seed extract, a valerian extract and a safflower seed extract.

The combination including the grape seed extract, the valerian extract and the safflower seed extract according to the present invention has effects of inhibiting osteoclast differentiation, enhancing osteoblast activity, improving estrogen receptor activity, and improving expression of IGF-1 and osteocalcin, thereby exerting excellent effects of preventing and alleviating female menopause symptoms. In addition, the present invention provides a food composition with excellent effects of preventing and alleviating female menopause symptoms, which contains, as an active ingredient, a combination consisting of natural extracts that are non-toxic, have no side effects and are harmless to the human body.

## Description

### [Technical Field]

The present invention relates to a food composition for preventing and alleviating female menopause symptoms containing a grape seed extract, a valerian extract and a safflower seed extract.

### [Background Art]

As women become older, ovarian function is deteriorated, thus resulting in a great reduction in the secretion of estrogen. Accordingly, women undergo decreases in response to follicle-stimulating hormone (FSH) and luteinizing hormone (LH) as well as in progesterone production. In particular, there is a high possibility that, around their fifties, women undergo menopause which is the time when the function of the ovaries completely ceases. From the beginning of menopause, the hormone balance is broken at the postmenopausal period, which causes various symptoms. This is called "climacterium". In addition, early menopause caused by ovariectomy, hysterectomy and the like may also result in climacteric symptoms.

That is, female menopause is a type of endocrine syndrome, which means the time when estrogen, which is a female hormone, is decreased due to aging of ovarian function, and physiological and sexual functions are decreased or lost. Symptoms of this climacterium include symptoms caused by vascular changes such as facial flushing, tachycardia, sweating or headache, symptoms resulting from musculoskeletal changes such as myalgia, arthralgia and back pain, symptoms caused by urinogenital changes such as urinary frequency or urinary incontinence, and symptoms due to brain-nervous system changes such as hypomnesis, depression, loss of concentration and dizziness. In addition, symptoms such as loss of vision and changes in skin and hair occur, and osteoporosis caused by hormone changes and diseases fatal to women's health such as cardiovascular diseases occur.

The recent development of civilization and the progress of science have led to the prolongation of the life expectancy of humans who live in modern times. Since the average life expectancy of women is 80.4 years old in 2002, for 30 to 40 years after 40s when menopausal symptoms slowly start to develop, special attention needs to be paid to the healthy life of women.

Because estrogen deficiency is a major cause of menopausal symptoms, estrogen replacement therapy has been used primarily as a remedy to improve women's menopausal symptoms. However, only 35 to 40% of women with menopausal symptoms use estrogen replacement therapy, and even they are reluctant to continuously use this method. (Ann. Intern. Med. 1999. 130:545-553). Estrogen replacement therapy is based on administration of artificial hormones, thus resulting in rejection response and side effects. A number of studies have been reported that adverse effects of estrogen replacement therapy may increase the risk of uterine bleeding, stroke, heart attack, breast cancer, and uterine cancer (Obstet. Gynecol. 1998. 91:678-684, New Engl. J. Med. 1995. 332:1589-1593).

Due to these problems, there is increasing interest in replacing estrogen therapy with a natural method of ingesting food, there is a need to develop agents for the treatment of menopausal symptoms, which are highly effective for mitigating climacteric symptoms without causing side effects and a great number of studies are thus underway to find natural vegetable ingredients such as vegetable estrogen as an alternative to synthetic estrogen.

### [Disclosure]

### [Technical Problem]

Therefore, the present invention has been made in view of the above problems, and it is an object of the present invention to provide a food composition containing a grape seed extract, a valerian extract and a safflower seed extract for preventing and alleviating female menopause symptoms.

### [Technical Solution]

In accordance with the present invention, the above and other objects can be accomplished by the provision of a food composition for preventing and alleviating female menopause symptoms, including, as an active ingredient, a combination of a grape seed extract, a valerian extract and a safflower seed extract.

Regarding the food composition for preventing and alleviating female menopause symptoms according to the present invention, the combination preferably contains 10 to 40% by weight of the grape seed extract, 15 to 50% by weight of the valerian extract and 30 to 70% by weight of the safflower seed extract. When the range is satisfied, the food composition can exhibit effects of improving estrogen receptor activity, inhibiting osteoclast differentiation, enhancing osteoblast activity, and improving expression of IGF-1 and osteocalcin. In addition, the combination of a grape seed extract, a valerian extract and a safflower seed extract can exhibit synergistic effects of preventing and alleviating female menopause symptoms, as compared to a single extract. In climacterium, the depletion of follicles in the ovaries leads to a decrease in the secretion of estrogen and an increase in the conversion of androgen to estrogen in peripheral tissues. It is also characterized by a decrease in estrogen levels and an increase in the secretion of follicle stimulating hormone and luteinizing hormone by the negative feedback mechanism of the hypothalamic-pituitary axis. Estrogen signal transfer is mediated by estrogen receptors which include estrogen receptor-α disposed at the nucleus (estrogen receptor-α), estrogen receptor-β and GPR30 (G protein-coupled receptor 30) disposed at the cell membrane.

The distribution of estrogen receptors depends on tissue and estrogen receptors are distributed in a variety of tissues. Accordingly, the decrease of estrogen in the climacterium causes symptoms in a variety of tissues where estrogen receptors are distributed. Most functional ingredients for alleviating female climacteric symptoms include vegetable estrogen ingredients which have a similar structure to estrogen and thus bind to estrogen receptors to provide estrogen activity.

As such, in order to provide effects of preventing and alleviating female menopause symptoms, there is a need for development of foods that are effective in improving estrogen receptor activity, are nontoxic and contain a great amount of vegetable estrogen.

Accordingly, as a result of research on food ingredients that are effective in improving expression of estrogen receptors from natural substances that are non-toxic and have no side effects, the optimal mix ratio of the grape seed extract, the valerian extract and the safflower seed extract is obtained, and a combination consisting of 10 to 40% by weight of the grape seed extract, 15 to 50% by weight of the valerian extract and 30 to 70% by weight of the safflower seed extract is highly effective for preventing and alleviating female menopause symptoms.

Regarding the food composition for preventing and alleviating female menopause symptoms according to the present invention, the grape seed extract, the valerian extract and the safflower seed extract are preferably extracted with, as an extraction solvent, water, C1-C4 lower alcohol or a mixture thereof, more preferably, water or 70% ethanol or a mixture thereof.

Regarding the food composition for preventing and alleviating female menopause symptoms according to the present invention, the extraction of the grape seed extract, the valerian extract and the safflower seed extract is, for example, hot water extraction, cold extraction, reflux extraction or ultrasonic extraction.

Regarding the food composition for preventing and alleviating female menopause symptoms according to the present invention, the extract preferably includes a concentrate obtained by extraction and then filtering and concentration under reduced pressure or vacuum. The extract is preferably a powder obtained by freeze drying, hot-air drying or spray drying.

Regarding the food composition for preventing and alleviating female menopause symptoms according to the present invention, the combination is preferably present in an amount of 0.01 to 50% by weight, with respect to the total weight of the food composition. When the combination is present in an amount of less than 0.01% by weight, the effect of preventing and alleviating female menopause symptoms is insufficient, and when the combination is present in an amount of higher than 50% by weight, the effects are low as compared to used amounts, thus resulting in low economic efficiency.

The food composition for preventing and alleviating female menopause symptoms according to the present invention preferably includes any one selected from meat, cereals, caffeinated beverages, regular beverages, chocolate, bread, snacks, confectionery, pizza, jelly, noodles, gums, ice cream, vitamin complexes and other health food supplements, but the present invention is not necessarily limited thereto.

The food composition for preventing and alleviating female menopause symptoms according to the present invention preferably further includes carriers, excipients and diluents commonly used for production of food. Examples of these ingredients include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, honey, methyl hydroxybenzoate, propyl hydroxybenzoate, magnesium stearate, and mineral oil.

The formulation is prepared using one or more diluents or excipients selected from commonly used fillers, thickeners, binders, wetting agents, disintegrants and surfactants.

Meanwhile, solid formulations for oral administration include one or more selected from tablets, pills, powders, granules and capsules, and these solid formulations are, for example, prepared by mixing one or more excipients selected from starch, calcium carbonate, sucrose or lactose, and gelatin. In addition, apart from a simple excipient, a lubricant such as magnesium stearate may be used.

In addition, liquid formulations for oral administration may include one or more selected from suspensions, liquids, emulsions and syrups, and may further include a variety of excipients such as humectants, sweeteners, perfumes and supplements, in addition to, as the simple diluent, water and liquid paraffin.

The food composition having the effects of preventing and alleviating female menopause symptoms according to the present invention may further include an additive which, for example, includes one or more selected from nutrients, vitamins, minerals (electrolytes), synthetic flavors and natural flavors, colorants and fillers (cheese, chocolate), pectic acid and salts thereof, alginic acid and salts thereof, organic acids, protective colloidal thickeners, pH adjusters, stabilizers, preservatives, glycerin, alcohols, and carbonates used in carbonated beverages. Further, the food composition may include flesh for preparing natural fruit juices, fruit drinks and fruit beverages. These ingredients may be used alone or in combination thereof. The additive is generally present in an amount of 0 to 50 parts by weight, with respect to 100 parts by weight of the combination, but the present invention is not limited thereto.

Regarding the food composition having the effects of preventing and alleviating female menopause symptoms, "female menopause symptoms" mean symptoms caused by decrease in hormone production in menopausal females, and climacteric or menopausal symptoms, for example, include one or more selected from facial flushing, sweating, sweating during sleep, dry skin, vaginal dryness, vaginal atrophy, lower urethral atrophy, dyspareunia, vaginitis, cystitis, dysuria, urgent micturition, loss of concentration, short-term amnestic disorders, anxiety, nervousness, decreased memory, hypoactive sexual desire disorder, myalgia, arthralgia and osteoporosis.

Meanwhile, grape (*Vitis vinifera* L) is a vine fruit tree which is the most widely cultivated vine in the world, belongs to *Rhamnales, Vitaceae,* and is classified into European grapes and American grapes. European grapes contain a great amount of carbohydrates and include glucose as main sugar and a small amount of other sugars. In addition, European grapes contain small amounts of vitamins B and C. Fruit juices thereof contain tartaric acid, malic acid, citric acid, racemic acid and salicylic acid. Grapes have stomachic, diuretic, tonic and thirst-quenching effects. Grape seeds, which account for 3 to 5% of grape weight, are reported to contain biologically active substances such as phenolic compounds and catechin as by-products after processing. In particular, as research on the active oxygen scavenging of catechins have recently been reported, interest in their use as natural antioxidants is increasing.

Meanwhile, valerian (*Valeriana officinalis*), which is called "Valeriana fauriei Briquet", is a perennial flowering plant that belongs to Valerianoideae, and produces dark blue leaves having a height of about 1 to 2 meters and white or pink flowers. Valerian is commonly found in Europe and northern Asia and it is known that it has already been used as an herb from ancient times and its roots have been used since BC. The root and rhizome extracts of this plant are known to exhibit various effects and representative examples of the effects include sleep aid, sedative effect, anxiolytic effect, anti-convulsant effect and pain relief.

Meanwhile, safflower (*Carthamus tinctoris*) is an annual plant belonging to the Asteraceae family, which has been cultivated primarily for medicinal applications in Korea, Japan and China. Safflower seeds have been reported to exhibit a decrease of cholesterol in the blood owing to high amounts of fats, especially, linoleic acid.

### [Advantageous Effects]

The combination including the grape seed extract, the valerian extract and the safflower seed extract according to the present invention has effects of inhibiting osteoclast differentiation, enhancing osteoblast activity, improving estrogen receptor activity, and improving expression of IGF-1 and osteocalcin, thereby exerting excellent effects of preventing and alleviating female menopause symptoms. In addition, the present invention provides a food composition with excellent effects of preventing and alleviating female menopause symptoms, which contains, as an active ingredient, a combination consisting of natural extracts that are non-toxic, have no side effects and are harmless to the human body.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 is a graph showing results of TRAP measurement tests to identify effects of a combination including a grape seed extract, a valerian extract and a safflower seed extract according to the present invention on formation of osteoclasts (*P*<0.05);
FIG. 2 is a graph showing results of RANKL tests to identify effects of the combination including a grape seed extract, a valerian extract and a safflower seed extract according to the present invention on formation of osteoclasts (*P*<0.05);
FIG. 3 is a graph showing results of alkaline phosphatase (ALP) activity measurement tests to identify effects of the combination including a grape seed extract, a valerian extract and a safflower seed extract according to the present invention on osteoblast activity (*P*<0.05);
FIG. 4 is a graph showing results of alkaline phosphatase (ALP) expression measurement tests to identify effects of the combination including a grape seed extract, a valerian extract and a safflower seed extract according to the present invention on osteoblast activity (*P*<0.05);
FIG. 5 is a graph showing results of tests to identify effects of the combination including a grape seed extract, a valerian extract and a safflower seed extract according to the present invention on IGF-1 gene expression;
FIG. 6 is a graph showing results of tests to identify effects of the combination including a grape seed extract, a valerian extract and a safflower seed extract according to the present invention on estrogen receptor expression (*P*<0.05); and
FIG. 7 is a graph showing results of tests to identify effects of the combination including a grape seed extract, a valerian extract and a safflower seed extract according to the present invention on osteocalcin expression (*P*<0.05).

### [Best Mode]

Hereinafter, the present invention will be described in more detail with reference to the following Example. The scope of the present invention is not limited to the following Example, and includes modifications of the technical concept equivalent thereto.

### [Preparation Example 1: Preparation of grape seed extract]

1 kg of a grape seed was dried and ground, 300 g of the resulting powder was placed in a gas collecting bottle, 1.8 L of 70% ethyl alcohol was added thereto, and extraction was repeatedly conducted at 85°C for three hours twice to obtain a grape seed extract. Then, the extract was filtered under reduced pressure through filter paper (Whatman No. 1, England), concentrated under reduced pressure at 60°C in a vacuum rotary evaporator and spray dried to prepare a grape seed extract.

### [Preparation Example 2: Preparation of valerian extract]

1 kg of a valerian root was dried and ground, 300 g of the resulting powder was placed in a gas collecting bottle, 1.5 L of 70% ethyl alcohol was added thereto, and extraction was repeatedly conducted at 70°C for 6 hours three times to obtain a valerian extract. Then, the extract was filtered under reduced pressure through filter paper (Whatman No. 1, England), concentrated under reduced pressure at 60°C in a vacuum rotary evaporator and spray dried to prepare a valerian extract.

### [Preparation Example 3: Preparation of safflower seed extract]

1 kg of a safflower seed was dried and ground, 300 g of the resulting powder was placed in a gas collecting bottle, 3 L of water was added thereto, and extraction was repeatedly conducted at 90°C for 6 hours three times to obtain a safflower seed extract. Then, the extract was filtered under reduced pressure through filter paper (Whatman No. 1, England), concentrated under reduced pressure at 60°C in a vacuum rotary evaporator and spray dried to prepare a safflower seed extract.

### [Examples 1 to 4: Preparation of combinations]

The grape seed extract, valerian extract, and safflower seed extract prepared in Preparation Examples 1 to 3 were mixed in different weight ratios shown in the following Table 1 to prepare combinations.

**TABLE 1**

| | Grape seed extract (% by weight) | Valerian extract (% by weight) | Safflower seed extract (% by weight) |
|---|---|---|---|
| Example 1 | 20 | 30 | 50 |
| Example 2 | 30 | 30 | 40 |
| Example 3 | 10 | 50 | 30 |
| Example 4 | 30 | 40 | 30 |

### [Test Example 1: Effects of combination on tartrate-resistant acid phosphatase (TRAP) activity, indicator of osteoporosis]

In the present Test Example, effects of the single extracts prepared in Preparation Examples 1 to 3, and the extract combinations prepared in Examples 1 to 4 on TRAP (tartrate-resistant acid phosphatase) activity were identified.

Tartrate-resistant acid phosphatase (TRAP) is an indicator of osteoclasts. Low activity of TRAP means inhibition of osteoclast differentiation.

In order to differentiate MC3T3-E1 cells into osteoclasts, femoral bone marrow cells were extracted from 7 week-old C57BL/6 female mice and cultured together with MC3T3-E1 cells in MEM (minimum essential medium supplemented with 10% FBS, 100 U/ml penicillin and 100 g/ml streptomycin).

MC3T3-E1 cells (1.5×10⁴ cells/well) and bone marrow cells (5×10⁵ cells/well) were seeded onto a 24-well plate and the MEM culture medium was treated with 10⁻⁸ M 1,25(OH)₂ vitamin D₃, 10⁻⁷ M dexamethasone and concentrations of 50 pg/ml of the samples (preparation Examples 1 to 3, and Examples 1 to 4). The culture medium was replaced every three days and the cells were cultured for seven days.

After culture for seven days, the cells were washed with phosphate-buffered saline (PBS) twice, collected by scraping with lysis buffer and were subjected to sonication at an interval of 10 seconds four times to obtain a cell lysate.

TRAP activity measurement kit used herein was an ELISA kit produced by Lifespan Biosciences and TRAP activity was measured at 450 nm in accordance with the manufacturer's instructions.

A group not treated with 1,25(OH)₂ vitamin D₃ was designated as a general group, and a group treated with 1,25(OH)₂ vitamin D₃ and not treated with a sample was designated as a control group. Results are shown in the following Table 2. A significant difference between test groups was obtained by ANOVA analysis and p<0.05 was determined to be significant difference.

**TABLE 2**

| | TRAP (% of control) |
|---|---|
| General group | 11±11.4^{d} |
| Control group | 100.2±2.86^{a} |
| Example 1 | 81.6±4.51^{c} |
| Example 2 | 82.2±3.03^{c} |
| Example 3 | 89.6±4.28^{b} |
| Example 4 | 85.4±2.61^{bc} |
| Preparation Example 1 | 93.6±3.43^{ab} |
| Preparation Example 2 | 97.8±2.77^{a} |
| Preparation Example 3 | 94.6±2.61^{a} |

Test results showed that the combination including the grape seed extract, the valerian extract and the safflower seed extract exhibited lower TRAP activity than single extracts (grape seed extract, valerian extract and safflower seed extract). In addition, among the combinations, Example 1 (20% by weight of the grape seed extract, 30% by weight of the valerian extract and 50% by weight of the safflower seed extract) exhibited the lowest TRAP activity.

In the following Test Example, testing was conducted using Example 1 that was found to have the highest effects.

### [Test Example 2: Effects of combination concentration on tartrate-resistant acid phosphatase (TRAP) activity, indicator of osteoporosis]

In the present Test Example, the effect of combination concentration on TRAP activity was identified.

MC3T3-E1 cells (1.5×10⁴ cells/well) and bone marrow cells (5×10⁵ cells/well) were seeded onto a 24 well plate and the MEM culture medium was treated with 10⁻⁸ M 1,25(OH)₂ vitamin D₃, 10⁻⁷ M dexamethasone, and 0, 20, 50 and 100 pg/ml of the sample (Example 1). The culture medium was replaced every three days and the cells were cultured for seven days.

After culture for seven days, the cells were washed with phosphate-buffered saline (PBS) twice, collected by scraping with lysis buffer, and were subjected to sonication at an interval of 10 seconds four times to obtain a cell lysate.

The TRAP activity measurement kit used herein was an ELISA kit produced by Lifespan Biosciences and TRAP activity was measured at 450 nm in accordance with the manufacturer's instructions. A group not treated with 1,25(OH)₂ vitamin D₃ was designated as a general group, and a group not treated with the composition was designated as a control group.

Test results showed that, when the combination (Example 1) was treated at concentrations of 50 pg/ml and 100 pg/ml, TRAP activity was significantly inhibited concentration-dependently.

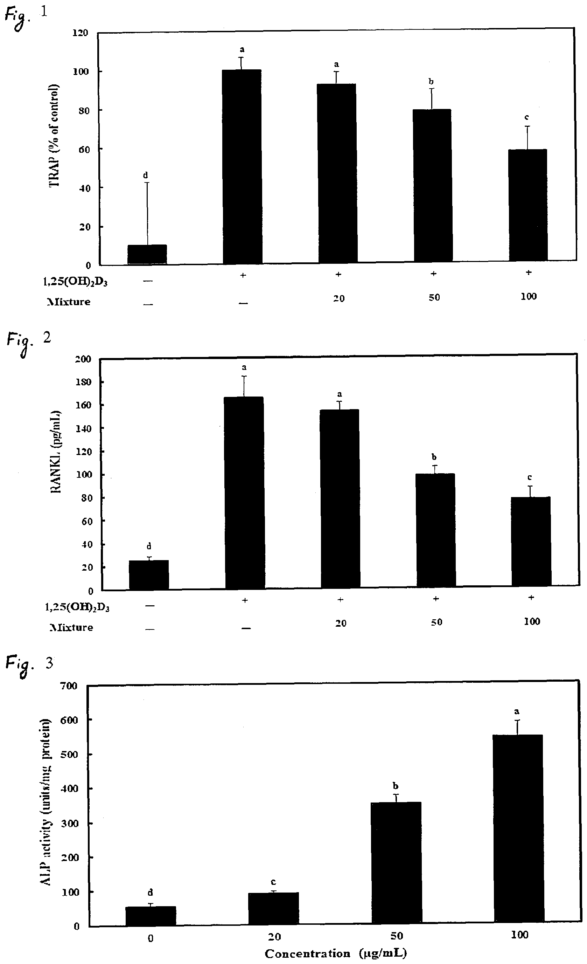

These results showed that, depending on the concentration of the combination, the effect of inhibiting osteoclast differentiation was improved (FIG. 1). FIG. 1 is a graph showing results of TRAP measurement tests to identify effects of the combination (Example 1) including a grape seed extract, a valerian extract and a safflower seed extract according to the present invention on formation of osteoclasts (*P*<0.05).

### [Test Example 3: Identification of effects of combination on osteoclast differentiation (measurement of RANKL expression)]

In the present Test Example, the amount of RANKL (receptor activator of NF-κB ligand) expressed was measured in order to identify the effect of inhibiting osteoclast differentiation by the combination.

RANKL, which is part of the TNF family, is known to be activated by NF-κB and MAPKs (mitogen-activated kinase) in osteoclast differentiation (J. Bone Miner Metab. 2014. 32: 598-604) and to act as a marker of osteoclast differentiation.

MC3T3-E1 cells (1.5×10⁴ cells/well) and bone marrow cells (5×10⁵ cells/well) were seeded onto a 24 well plate and the MEM culture medium was treated with 10⁻⁸ M 1,25(OH)₂ vitamin D₃, 10⁻⁷ M dexamethasone and 0, 20, 50 and 100 pg/ml of the sample (Example 1). The culture medium was replaced every three days and the cells were cultured for seven days.

After culture for seven days, the media of cells were removed and testing was conducted in accordance with the manufacturer's instructions using Quantikine M mouse RANKL immunoassay kit (R&D System, USA). Each well was measured at 450 nm within 40 minutes using an ELISA reader. A group not treated with 1,25(OH)₂ vitamin D₃ was designated as a general group, and a group not treated with the composition was designated as a control group.

Test results showed that, when the combination (Example 1) was treated at concentrations of 50 pg/ml and 100 pg/ml, the amount of expressed RANKL was significantly decreased concentration-dependently.

It could be confirmed from these results that the combination had the effect of inhibiting osteoclast differentiation (FIG. 2). FIG. 2 is a graph showing results of RANKL measurement tests to identify effects of the combination (Example 1) including a grape seed extract, a valerian extract and a safflower seed extract according to the present invention on formation of osteoclasts (*P*<0.05).

### [Test Example 4: Measurement of alkaline phosphatase (ALP) activity]

Osteoblasts have alkaline phosphatase (ALP) on the cell membranes, and such an enzyme is known to be an active marker of osteoblasts, which is found at high concentrations in the outer membrane of the cell and calcified tissues, and regulates transport, cell division and differentiation during the calcification process (Int Rev Cytol. 1995. 265-358).

Accordingly, in the present Test Example, activity of alkaline phosphatase (ALP) was measured in order to identify effects of the combination on osteoblast activity.

For measurement of ALP activity, MC3T3-E1 cell lines were treated with a MEM culture medium, 10 mM beta-glycerophosphate and 50 pg/ml ascorbic acid for 6 days, to differentiate the cells into osteoblasts. The differentiated cells were seeded onto a 24 well plate, treated with the combination (Example 1) at 0, 20, 50 and 100 pg/ml and cultured for 3 days.

After culture, the cells were washed with phosphate-buffered saline (PBS) twice, collected by scraping with lysis buffer and subjected to sonication at an interval of 10 seconds four times. 1 ml of a reaction mixture (alkaline buffer:stock substrate, 1:1) was added to each well and incubated at 37°C for 30 minutes. For measurement, testing was conducted in accordance with the Abcam protocol. Finally, 12L of 1N NaOH was added to each well and absorbance was measured at 405 nm using an ELISA reader (VERSAMAXSL-20 Molecular Devices, Seoul, Korea).

Test results showed that, as compared to the control group not treated with the combination, cells treated with the combination at different concentrations, exhibited superior ALP activity, in particular, concentration-dependently exhibited high ALP activity when treated at 20, 50 and 100 pg/ml.

These results showed that the combination had the effect of promoting osteoblast activity (FIG. 3). FIG. 3 is a graph showing results of alkaline phosphatase (ALP) activity measurement tests to identify effects of the combination (Example 1) including a grape seed extract, a valerian extract and a safflower seed extract according to the present invention on osteoblast activity (*P*<0.05).

### [Test Example 5: Effects of combination on gene expression of alkaline phosphatase (ALP), IGF-1, estrogen receptor and osteocalcin]

IGF-1 (insulin-like growth factor-1) is a growth factor closely related to growth hormone and is secreted from the pituitary gland stimulated by growth hormones. IGF-1 is known to function to promote the growth and differentiation of cartilage cells in cartilage tissues and to promote bone skeletal growth (Nat Med. 2003. 9:1033-1038).

Estrogen receptors are a group of proteins, which are present in the nucleus of respective tissues or in the cell thereof, that receive estrogen and conduct estrogen signaling. Therefore, estrogen decreases during climacterium and symptoms appear in various tissues where estrogen receptors are distributed. Estrogenic activity of the combination is evaluated by measuring the degree of gene expression of estrogen receptors.

Osteocalcin is synthesized by osteoblasts and used as an indicator of bone formation. That is, the concentration of osteocalcin in the serum directly reflects the activity of the osteoblast.

Accordingly, in the present Test Example, real-time PCR was conducted in order to identify effects of combination on gene expression of alkaline phosphatase (ALP), IGF-1, estrogen receptor and osteocalcin.

MC3T3-E1 cell lines were treated with a MEM culture medium, 10 mM beta-glycerophosphate and 50 pg/ml ascorbic acid for 6 days, to differentiate the cells into osteoblasts. The differentiated cells were seeded onto a 24 well plate, treated with the combination (Example 1) at 0, 20, 50 and 100 pg/ml and cultured for 3 days.

The cells cultured for 3 days were collected with 0.25% trypsin-EDTA, total RNA was extracted using an RNeasy extraction kit (QIAGEN, USA) in accordance with the manufacturer's instructions, for synthesis of cDNA, 4 µL of 5×iScript select reaction mix, 2 µL of an oligo (dT) primer set, 5 µL of a RNA sample and 8 µL of nuclease free water were added to 5 µg of total RNA using iScript™ Select cDNA Synthesis Kit (BIO-RAD, USA), and finally, 1 µL of iScript reverse transcriptase was added thereto and the mixture was thoroughly mixed by shaking up and down with a pipette.

The reaction was conducted at 42°C for 60 minutes and then at 85°C for 5 minutes, and the synthesized cDNAs were then used for PCR reaction. A Step One Real-Time PCR system (Applied Biosystems, USA) was used for real-time PCR and the reaction was conducted in accordance with the method of iQ SYBR Green Supermix (BIO-RAD, USA).

The final composition of the mixture for PCR was 2 µL (10 to 100 ng) of cDNA, 10 µL of 2×iQ SYBR Green Supermix, 1 µL (250 nM) of each forward and reverse primers, and 7 µL of H₂O. After hot start at 95°C for 10 minutes, PCR was conducted by 40 cycling at 95°C for 15 seconds, at 55°C for 15 seconds and at 72°C for 30 seconds, and finally finishing process at 95°C for 15 seconds, at 60°C for 1 minute and at 95°C for 15 seconds. Primers used for the reactions are shown in the following Table 3.

**TABLE 3**

| | | |
|---|---|---|
| ALP | Forward | 5'-AAC CCA GAC ACA AGC ATT CC-3' |
| | Reverse | 5'-GAG ACA TTT TCC CGT TCA CC-3' |
| IGF-1 | Forward | 5'-ABA CAG GCA TTG TGG ATG AG-3' |
| | Reverse | 5'-TGA GTC TTG GGC ATG TCA GT-3' |
| Estrogen receptor | Forward | 5'-GTC TCC GAG CCC GCT GAT GCT ACT GCA C-3' |
| | Reverse | 5'-CGG ATG CCC CTC CAC GGC TAG TGG-3' |
| Osteocalcin | Forward | 5'-GCA ATA AGG TAG TGA ACA GAC TCC-3' |
| | Reverse | 5'-GTT TGT AGG CGG TCT TCA AGC-3' |

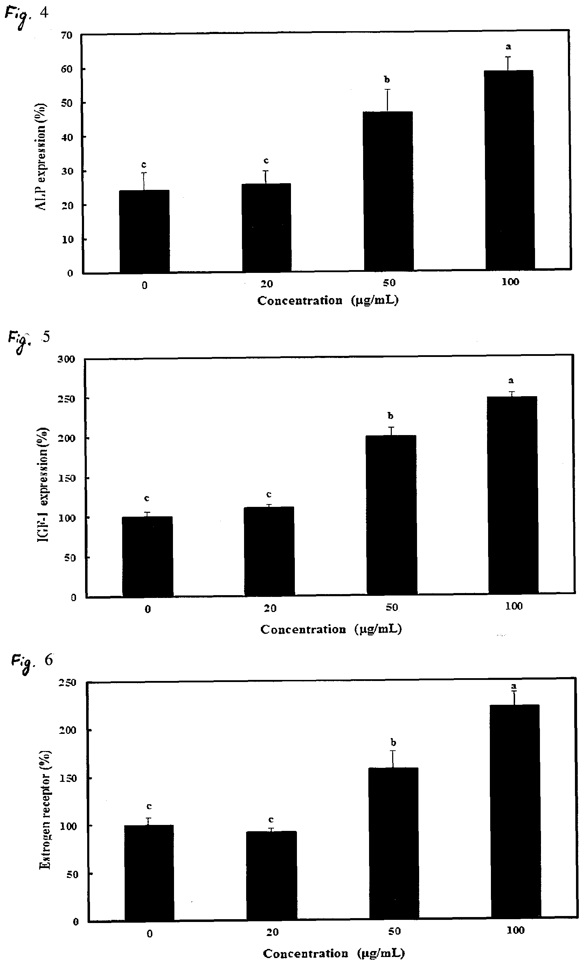

Measurement results of alkaline phosphatase (ALP) expression showed that, as compared to the control group, cells treated with the combination at 50 pg/ml and 100 pg/ml showed a concentration-dependently significant increase in alkaline phosphatase (ALP) (FIG. 4). FIG. 4 is a graph showing results of alkaline phosphatase (ALP) expression measurement tests to identify effects of the combination (Example 1) including a grape seed extract, a valerian extract and a safflower seed extract according to the present invention on expression of osteoblast activity (*P*<0.05)

Measurement results of IGF-1 expression showed that, as compared to the control group, cells treated with the combination at 50 pg/ml and 100 pg/ml showed a concentration-dependently significant increase in IGF-1 gene expression (FIG. 5). FIG. 5 is a graph showing results of tests to identify effects of the combination (Example 1) including a grape seed extract, a valerian extract and a safflower seed extract according to the present invention on IGF-1 gene expression.

Measurement results of estrogen receptor expression showed that, as compared to the control group, cells treated with the combination at 50 pg/ml and 100 pg/ml showed a concentration-dependently significant increase in estrogen receptor expression (FIG. 6). FIG. 6 is a graph showing results of estrogen receptor expression measurement tests to identify effects of the combination (Example 1) including a grape seed extract, a valerian extract and a safflower seed extract according to the present invention on estrogen receptor expression (*P*<0.05).

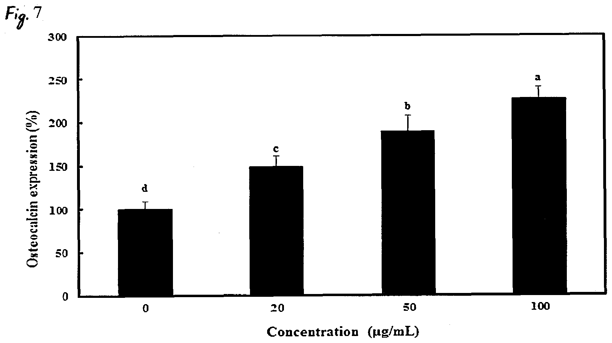

Measurement results of osteocalcin expression showed that, as compared to the control group, cells treated with the combination at 20 pg/ml, 50 pg/ml and 100 pg/ml showed a concentration-dependently significant increase in osteocalcin expression (FIG. 7). FIG. 7 is a graph showing results of tests to identify effects of the combination (Example 1) including a grape seed extract, a valerian extract and a safflower seed extract according to the present invention on osteocalcin expression (*P*<0.05).

It could be confirmed from these results that the combination had an excellent effect of enhancing osteoblast activity and superior estrogen activity.

### [Example 5: Preparation of food composition having effects of preventing and alleviating female menopause symptoms]

In the present Example, a food composition having effects of preventing and alleviating female menopause symptoms was prepared.

### (1) Preparation of Sunsik (Korean traditional grain powder mixture)

Brown rice, barley, glutinous rice and adlay were gelatinized (alpha-converted), dried, distributed and prepared as a powder with a particle size of 60 mesh using a grinder. Black beans, black sesame and perilla were steamed by a well-known method, dried, distributed and ground to prepare a powder with a particle size of 60 mesh. Then, 30% by weight of brown rice, 15% by weight of adlay, 20% by weight of barley, 9% by weight of glutinous rice, 7% by weight of perilla, 8% by weight of black bean, 7% by weight of black sesame, 3% by weight of the combination including the grape seed extract, the valerian extract and the safflower seed extract according to the present invention (Example 1), 0.5% by weight of lacquer top and 0.5% by weight of rehmannia were mixed to prepare Sunsik.

### (2) Preparation of chewing gum

20% by weight of a gum base, 76.9% by weight of sugar, 1% by weight of a flavor, 2% by weight of water and 0.1% by weight of the combination including the grape seed extract, the valerian extract and the safflower seed extract according to the present invention (Example 1) were mixed to prepare a chewing gum by a conventional method.

### (3) Preparation of candy

60% by weight of sugar, 39.8% by weight of starch syrup, 0.1% by weight of a flavor and 0.1% by weight of the combination including the grape seed extract, the valerian extract and the safflower seed extract according to the present invention (Example 1) were mixed to prepare a candy by a conventional method.

### (4) Preparation of biscuit

25.59% by weight of 1^{st} class weak flour, 22.22% by weight of 1^{st} class medium flour, 4.80% by weight of refined sugar, 0.73% by weight of table salt, 0.78% by weight of glucose, 11.78% by weight of palm shortening, 1.54% by weight of ammonium, 0.17% by weight of sodium bicarbonate, 0.16% by weight of sodium bisulfite, 1.45% by weight of rice flour, 0.0001% by weight of vitamin B1, 0.0001% by weight of vitamin B2, 0.04% by weight of milk flavoring, 20.6998% by weight of water, 1.16% by weight of whole milk powder, 0.29% by weight of modified milk powder, 0.03% by weight of calcium phosphate, 0.29% by weight of a spray salt, 7.27% by weight of spray milk, and 1% by weight of the combination including the grape seed extract, the valerian extract and the safflower seed extract according to the present invention (Example 1) were mixed to prepare a biscuit by a conventional method.

### (5) Preparation of healthy beverage

0.26% by weight of honey, 0.0002% by weight of thioctic acid amide, 0.0004% by weight of nicotinamide, 0.0001% by weight of riboflavin sodium phosphate, 0.0001% by weight of pyridoxine HCl, 0.001% by weight of inositol, 0.002% by weight of orotic acid, 98.7362% by weight of water and 1% by weight of the combination including the grape seed extract, the valerian extract and the safflower seed extract according to the present invention (Example 1) were mixed to prepare a healthy beverage by a conventional method.

### (6) Preparation of sausage

65.18% by weight of pork, 25% by weight of chicken, 3.5% by weight of starch, 1.7% by weight of soybean protein, 1.62% by weight of table salt, 0.5% by weight of glucose and 1.5% by weight of glycerin, and 1% by weight of the combination including the grape seed extract, the valerian extract and the safflower seed extract according to the present invention (Example 1) were mixed to prepare a sausage by a conventional method.

### (7) Preparation of health supplement food

50% by weight of the combination including the grape seed extract, the valerian extract and the safflower seed extract according to the present invention (Example 1), 16% by weight of guar gum hydrolysate, 0.01% by weight of vitamin B hydrochloride, 0.01% by weight of vitamin B6 hydrochloride, 0.23% by weight of DL-methionine, 0.7% by weight of magnesium stearate, 31.2% by weight of lactose and 1.85% by weight of corn starch were mixed to prepare a tablet-type health supplement food by a conventional method.

## Claims

1. A food composition for preventing and alleviating female menopause symptoms comprising, as an active ingredient, a combination of a grape seed extract, a valerian extract and a safflower seed extract.

2. The food composition according to claim 1, wherein the combination is a combination comprising 10 to 40% by weight of the grape seed extract, 15 to 50% by weight of the valerian extract and 30 to 70% by weight of the safflower seed extract.

3. The food composition according to claim 1, wherein the grape seed extract, the valerian extract and the safflower seed extract are extracted with water, C1 to C4 lower alcohol or a mixture thereof as an extraction solvent.

4. The food composition according to claim 1, wherein the combination is present in an amount of 0.01 to 50% by weight with respect to the total weight of the food composition.
